# EUROPEAN PATENT SPECIFICATION

(11) **EP 3 122 788 B1**
(45) Date of publication and mention of the grant of the patent: **18.04.2018**
(21) Application number: 15713417.2
(22) Date of filing: 24.03.2015
(51) Int. Cl.: C08F 4/651, C08F 10/00

(54) **CATALYST COMPONENTS FOR THE POLYMERIZATION OF OLEFINS**
KATALYSATORKOMPONENTEN ZUR OLEFINPOLYMERISATION
COMPOSANTS CATALYTIQUES POUR LA POLYMÉRISATION D'OLÉFINES

(30) Priority: 26.03.2014 EP 14161750
(43) Date of publication of application: 01.02.2017
(73) Proprietor: Basell Poliolefine Italia S.r.l., 20121 Milano (IT)
(72) Inventor: MIGNOGNA, Alessandro, I-44122 Ferrara (IT); MORINI, Giampiero, I-44122 Ferrara (IT)
(74) Representative: Giberti, Stefano
(86) International application number: PCT/EP2015/056198
(87) International publication number: WO 2015/144668

(56) References cited:
- EP-A1- 2 712 875
- WO-A1-2014/013401
- WO-A2-2006/110234

## Description

### FIELD OF THE INVENTION

The present disclosure relates to catalyst components for the polymerization of olefins, in particular propylene, comprising a Mg dihalide based support on which are supported Ti atoms and an electron donor compound containing a carbonic ester and a carbamate function. The present disclosure further relates to the catalysts obtained from said components and to their use in processes for the polymerization of olefins in particular propylene.

### BACKGROUND OF THE INVENTION

Catalyst components for the stereospecific polymerization of olefins are widely known in the art. Concerning the polymerization of propylene, the most spread out catalyst family belongs to the Ziegler-Natta category which, in general terms, comprises a solid catalyst component, constituted by a magnesium dihalide on which are supported a titanium compound and an internal electron donor compound, used in combination with an Al-alkyl compound. Conventionally however, when a higher cristallinity of the polymer is required, also an external donor (for example an alkoxysilane) is needed in order to obtain higher isotacticity. One of the preferred classes of internal donors is constituted by the esters of phthalic acid, diisobutylphthalate being the most used. The phthalates are used as internal donors in combination with alkylalkoxysilanes as external donor. This catalyst system gives good performances in terms of activity, isotacticity and xylene insolubility.

One of the problems associated with the use of this catalyst system is that the phthalates have recently raised concerns due to the medical issues associated with their use and some compounds within this class have been classified as source of heavy health problems.

Consequently, research activities have been devoted to discover alternative classes of internal donors for use in the preparation of catalyst components for propylene polymerization.

Some of the tested catalysts contain donor structures having simultaneously amide groups and ester groups. WO2006/110234 describes amino acid derivatives including one carbamate group and one free ester function. The catalysts generated by these structures have very low activity and sterospecificity in bulk propylene polymerization (table 2).

Another class of internal donors is described in WO2011/068770 which relates to two atoms bridged dicarbonates compounds. According to this patent good results can be obtained when the diol portion is part of a substituted phenyl group. According to the applicant said catalysts show interesting stereospecificity but the activity is to be improved. Moreover, the said document shows that donors having only one carbonate function such as diethyl carbonate have very low performances.

### SUMMARY OF THE INVENTION

Surprisingly, the applicant has found that a class of donors containing only one carbonic ester function within a specific structure generates catalysts showing an excellent balance of activity and stereospecificity when also a carbamic function is present.

Accordingly, it is an object of the present disclosure a catalyst component for the polymerization of olefins comprising Mg, Ti and an electron donor of formula (I) 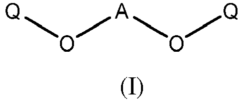 where each Q is a group -COOR¹ in which R¹ is selected from C₁-C₁₅ hydrocarbon groups, optionally containing a heteroatom selected from halogen, P, S, N,O; or a group -CON(R²)₂ in which R² groups, equal to or different from each other, are hydrogen or R¹ groups which can be fused together to form one or more cycles, and A is a bivalent bridging group with the proviso that Q groups cannot be simultaneously a group -COOR¹ or -CON(R²)₂.

### DETAILED DESCRIPTION OF THE INVENTION

Preferably, A is a bivalent bridging group with chain length between the two bridging bonds being 1-10 atoms. In case of cyclic structures acting as bridging groups the term "chain length" is referred to the shortest sequence of atoms bridging the oxygen atoms of formula (I). In a preferred general embodiment, the bridging group has formula -(ZR³m)n- in which, independently, Z is selected from C, Si, Ge, O, N, S or P, the R³ groups, equal to or different from each other, are hydrogen or a C1-C20 hydrocarbon radicals, optionally containing a heteroatom selected from halogen, P, S, N, O and Si, which can be fused together to form one or more cycles, m is a number satisfying the valences of Z and n is an integer ranging from 1 to 10. Preferably, in the bridging group of formula -(ZR3m)n- the atoms O, S, and N are not directly linked to the oxygen of formula (I), i.e. they are not the terminal atoms of the bridging group. Preferably, Z is selected from C and Si. In a more preferred embodiment, Z is carbon.

In a particular embodiment, the said bivalent bridging group is selected from the group consisting of aliphatic, alicyclic and aromatic bivalent radicals, optionally substituted with C₁-C₁₅ hydrocarbon groups and/or with heteroatoms selected from halogen, P, S, N, O and Si, and having a bridging chain length ranging from 1 to 6 atoms and especially from 1 to 4 atoms.

In a particularly preferred embodiment, the bridging group is an aliphatic or alicyclic bridging group having a bridging chain length of 1-6 carbon atoms. Among this class, particularly preferred bridging groups are those of formula -(CR⁴ₚ)ₛ- in which R⁴ is independently hydrogen or a C₁-C₂₀ hydrocarbon radicals, optionally substituted with heteroatoms selected from halogen, P, S, N, O and Si, which can be fused together to form one or more cycles, p is a number satisfying the available valence of carbon and s is a number from 1 to 6 preferably from 1 to 4. Examples of bridging groups are methyliden, ethane-1,2-diyl, butane-2,3-diyl, pentane-2,4-diyl, 2,2-diisobutylpropane-1,3-diyl, cyclohexane-1,2-diyl, cyclopentane - 1,2-diyl. The bridging group pentane-2,4-diyl being the most preferred.

Another class of preferred bridging group is the one based on aromatic groups which through the carbon ring atoms can link the two oxygen of formula (I). Among them, particularly preferred are the phenyl groups, optionally substituted with halogens or C₁-C₂₀ alkyl radicals, bridging the oxygen atoms in position 1,2 or 1,3 or 1,4 and the naphthalene groups, optionally substituted bridging the oxygen groups in position 1,2 or 2,3 or 1,8.

Particularly preferred internal donor structures are those of formula (II) below 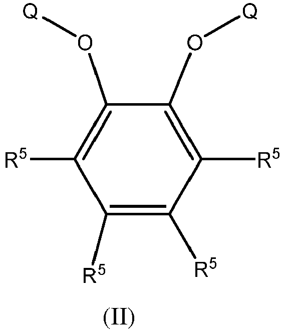 in which the Q groups have the same meaning previously specified, and R⁵, independently, is selected from hydrogen, halogens or C₁-C₁₅ hydrocarbon groups optionally substituted with heteroatoms selected from halogen, P, S, N, O and Si, with the proviso that at least one of R⁵ is different from hydrogen.

Preferred structures of formula (II) are those in which at least two of the R⁵ groups are different from hydrogen. More preferably the aromatic ring of formula (II) is substituted in position 3,5 and/or 6. In all these cases, R⁵ groups are preferably selected from C₁-C₅ alkyl groups. Particularly preferred is the substitution in position 3 and/or 6 with a primary alkyl group especially methyl, and in position 4 and/or 5 with a tertiary alkyl group especially tert-butyl.

Specific examples of aromatic bridging groups are 1,2-phenylene, 3-methyl-1,2-phenylene, 4-chloro-1,2-phenylene, 4-(*tert*-butyl)-1,2-phenylene, 3,6-dimethyl-1,2-phenylene, 3,5-dimethyl-1,2-phenylene, 5-(*tert*-butyl)-3-methyl-1,2-phenylene, 3,5-diisopropyl-1,2-phenylene. 5-(*tert*-butyl)-3-methyl-1,2-phenylene is the most preferred.

Preferably, in the formulas (I) and (II) the R¹ groups are independently selected from C₁-C₁₅ alkyl groups, C₆-C₁₄ aryl groups, C₃-C₁₅ cycloalkyl groups, and C₇-C₁₅ arylalkyl or alkylaryl groups; the same applies to R² groups which can additionally be hydrogen. More preferably, the R¹ groups in formulae (I) and (II) are preferably alkyl groups in particular C₁-C₅ alkyl groups.

Preferably , in the formulas (I) and (II) the R² groups are independently selected from hydrogen or C₁-C₁₀ alkyl groups and even more preferably from hydrogen or C₁-C₅ alkyl groups in particular ethyl.

Particularly preferred subgroups are those expressed by the formula (III) below 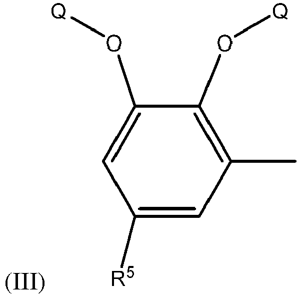 In which R⁵ is a branched alkyl group, preferably t-butyl.
In view of the fact that each of the Q groups can be selected from -COOR¹ and CON(R²)₂ but cannot be simultaneously a group -COOR¹ or -CON(R²)₂ its structure, the compounds of formula (III) can exist in two different isomeric forms.

Preferably, in formula (III) R¹ and R² are C₁-C₅ alkyl groups. Preferably they are chosen among linear C₁-C₅ alkyl groups in particular ethyl and n-propyl.

Preferably, the final amount of electron donor compound in the solid catalyst component ranges from 1 to 25% by weight preferably in the range from 3 to 20% by weight.

Examples of structures of formulas (I), (II) and (III) are the following:
(9-(((ethoxycarbonyl)oxy)methyl)-9H-fluoren-9-yl)methyl diethylcarbamate, 2-((ethoxycarbonyl)oxy)-3,6-dimethylphenyl diethylcarbamate, 2-((ethoxycarbonyl)oxy)-3,6-dimethylphenyl dimethylcarbamate, 2-((ethoxycarbonyl)oxy)-3,6-dimethylphenyl ethylcarbamate, 2-((ethoxycarbonyl)oxy)-3-methylphenyl diethylcarbamate, 2-((ethoxycarbonyl)oxy)-3-methylphenyl dimethylcarbamate, 2-((ethoxycarbonyl)oxy)-3-methylphenyl ethylcarbamate, 2-((ethoxycarbonyl)oxy)-4-methylphenyl diethylcarbamate, 2-((ethoxycarbonyl)oxy)-4-methylphenyl dimethylcarbamate, 2-((ethoxycarbonyl)oxy)-4-methylphenyl ethylcarbamate, 2-((ethoxycarbonyl)oxy)-5-methylphenyl diethylcarbamate, 2-((ethoxycarbonyl)oxy)-5-methylphenyl dimethylcarbamate, 2-((ethoxycarbonyl)oxy)-5-methylphenyl ethylcarbamate, 2-((ethoxycarbonyl)oxy)-6-methylphenyl dimethylcarbamate, 2-((ethoxycarbonyl)oxy)-6-methylphenyl ethylcarbamate, 2-((ethoxycarbonyl)oxy)phenyl diethylcarbamate, 2-((ethoxycarbonyl)oxy)phenyl dimethylcarbamate, 2-((ethoxycarbonyl)oxy)phenyl ethylcarbamate, 3-(tert-butyl)-6-((ethoxycarbonyl)oxy)-2,5-dimethylphenyl diethylcarbamate, 3-(tert-butyl)-6-((ethoxycarbonyl)oxy)-2,5-dimethylphenyl dimethylcarbamate, 3-(tert-butyl)-6-((ethoxycarbonyl)oxy)-2,5-dimethylphenyl ethylcarbamate, 4-((ethoxycarbonyl)oxy)pentan-2-yl diethylcarbamate, 4-(tert-butyl)-2-((ethoxycarbonyl)oxy)-3,6-dimethylphenyl diethylcarbamate, 4-(tert-butyl)-2-((ethoxycarbonyl)oxy)-3,6-dimethylphenyl dimethylcarbamate, 4-(tert-butyl)-2-((ethoxycarbonyl)oxy)-3,6-dimethylphenyl ethylcarbamate, 4-(tert-butyl)-2-((ethoxycarbonyl)oxy)-6-methylphenyl diethylcarbamate, 4-(tert-butyl)-2-((ethoxycarbonyl)oxy)-6-methylphenyl dimethylcarbamate, 4-(tert-butyl)-2-((ethoxycarbonyl)oxy)-6-methylphenyl dipropylcarbamate, 4-(tert-butyl)-2-((ethoxycarbonyl)oxy)-6-methylphenyl ethylcarbamate, 4-(tert-butyl)-2-((ethoxycarbonyl)oxy)phenyl diethylcarbamate, 4-(tert-butyl)-2-((ethoxycarbonyl)oxy)phenyl dimethylcarbamate, 4-(tert-butyl)-2-((ethoxycarbonyl)oxy)phenyl dipropylcarbamate, 4-(tert-butyl)-2-((ethoxycarbonyl)oxy)phenyl ethylcarbamate, 5-(tert-butyl)-2-((ethoxycarbonyl)oxy)-3-methylphenyl diethylcarbamate, 5-(tert-butyl)-2-((ethoxycarbonyl)oxy)-3-methylphenyl dimethylcarbamate, 5-(tert-butyl)-2-((ethoxycarbonyl)oxy)-3-methylphenyl dipropylcarbamate, 5-(tert-butyl)-2-((ethoxycarbonyl)oxy)-3-methylphenyl ethylcarbamate, 5-(tert-butyl)-2-((ethoxycarbonyl)oxy)phenyl diethylcarbamate, 5-(tert-butyl)-2-((ethoxycarbonyl)oxy)phenyl dimethylcarbamate, 5-(tert-butyl)-2-((ethoxycarbonyl)oxy)phenyl dipropylcarbamate, 5-(tert-butyl)-2-((ethoxycarbonyl)oxy)phenyl ethylcarbamate, 6-((ethoxycarbonyl)oxy)cyclohexa-1,2,3,5-tetraen-1-yl diethylcarbamate, 8-((ethoxycarbonyl)oxy)naphthalen-1-yl diethylcarbamate 2-((butoxycarbonyl)oxy)-4-(tert-butyl)-6-methylphenyl diethylcarbamate, 2-((butoxycarbonyl)oxy)-5-(tert-butyl)-3-methylphenyl diethylcarbamate, 4-(tert-butyl)-2-((isobutoxycarbonyl)oxy)-6-methylphenyl diethylcarbamate, 5-(tert-butyl)-2-((isobutoxycarbonyl)oxy)-3-methylphenyl diethylcarbamate, 2-((ethoxycarbonyl)oxy)-3-methylphenyl dipropylcarbamate, 2-((ethoxycarbonyl)oxy)-6-methylphenyl dipropylcarbamate. 4-(tert-butyl)-2-((ethoxycarbonyl)oxy)-6-methylphenyl diethylcarbamate and 5-(tert-butyl)-2-((ethoxycarbonyl)oxy)-3-methylphenyl diethylcarbamate are the most preferred.

The internal donors disclosed above can be generally prepared by reacting an excess of the starting diol HO-A-OH with a suitable carbamoyl chloride followed by removal of the unreacted diol by aqueous washings, distillation or chromatographic techniques. Then the monocarbamate-monoalcohol is reacted with a suitable chloroformate or carbonate. Both steps are carried out in presence of a base and their order can be inverted. Alternately, the internal donor can be prepared by converting the diol into a cyclic carbonate. This cyclic carbonate is reacted with a proper primary or secondary amine and the addition product reacted with a proper chloroformate or carbonate.

The amount of Ti atoms in the solid catalyst component is preferably higher than 1.5%wt more preferably higher than 2.0% with respect to the total weight of said catalyst component.

As explained above, the catalyst components of the disclosure comprise, in addition to the above electron donors, Ti, Mg and halogen. In particular, the catalyst components comprise a titanium compound, having at least a Ti-halogen bond and the above mentioned electron donor compounds supported on a Mg halide. The magnesium halide is preferably MgCl₂ in active form which is widely known from the patent literature as a support for Ziegler-Natta catalysts. Patents USP 4,298,718 and USP 4,495,338 were the first to describe the use of these compounds in Ziegler-Natta catalysis. It is known from these patents that the magnesium dihalides in active form used as support or co-support in components of catalysts for the polymerization of olefins are characterized by X-ray spectra in which the most intense diffraction line that appears in the spectrum of the non-active halide is diminished in intensity and is replaced by a halo whose maximum intensity is displaced towards lower angles relative to that of the more intense line.

The preferred titanium compounds used in the catalyst component of the present disclosure are TiCl₄ and TiCl₃; furthermore, also Ti-haloalcoholates of formula Ti(OR)_{m-y}X_{y} can be used, where m is the valence of titanium, y is a number between 1 and m-1, X is halogen and R is a hydrocarbon radical having from 1 to 10 carbon atoms.

The preparation of the solid catalyst component can be carried out according to several methods. One method comprises the reaction between magnesium alcoholates or chloroalcoholates (in particular chloroalcoholates prepared according to USP 4,220,554) and an excess of TiCl₄ in the presence of the electron donor compounds at a temperature of about 80 to 130°C.

According to a preferred method, the solid catalyst component can be prepared by reacting a titanium compound of formula Ti(OR)_{m-y}X_{y}, where m is the valence of titanium and y is a number between 1 and m, preferably TiCl₄, with a magnesium chloride deriving from an adduct of formula MgCl₂•pROH, where p is a number between 0.1 and 6, preferably from 2 to 3.5, and R is a hydrocarbon radical having 1-18 carbon atoms. The adduct can be suitably prepared in spherical form by mixing alcohol and magnesium chloride in the presence of an inert hydrocarbon immiscible with the adduct, operating under stirring conditions at the melting temperature of the adduct (100-130°C). Then, the emulsion is quickly quenched, thereby causing the solidification of the adduct in form of spherical particles. Examples of spherical adducts prepared according to this procedure are described in USP 4,399,054 and USP 4,469,648. The so obtained adduct can be directly reacted with Ti compound or it can be previously subjected to thermal controlled dealcoholation (80-130°C) so as to obtain an adduct in which the number of moles of alcohol is generally lower than 3, preferably between 0.1 and 2.5. The reaction with the Ti compound can be carried out by suspending the adduct (dealcoholated or as such) in cold TiCl₄ (generally 0°C); the mixture is heated up to 80-130°C and kept at this temperature for 0.5-2 hours. The treatment with TiCl₄ can be carried out one or more times. The electron donor compound is preferably added during the treatment with TiCl₄. The preparation of catalyst components in spherical form are described for example in European Patent Applications EP-A-395083, EP-A-553805, EP-A-553806, EPA601525 and WO98/44009.

The solid catalyst components obtained according to the above method show a surface area (by B.E.T. method) generally between 20 and 500 m²/g and preferably between 50 and 400 m²/g, and a total porosity (by B.E.T. method) higher than 0.2 cm³/g preferably between 0.2 and 0.6 cm³/g. The porosity (Hg method) due to pores with radius up to 10.000Å generally ranges from 0.3 to 1.5 cm³/g, preferably from 0.45 to 1 cm³/g.

The solid catalyst component has an average particle size ranging from 5 to 120 µm and more preferably from 10 to 100 µm.

In any of these preparation methods the desired electron donor compounds can be added as such or, in an alternative way, it can be obtained *in situ* by using an appropriate precursor capable to be transformed in the desired electron donor compound by means, for example, of known chemical reactions.

Regardless of the preparation method used, the final amount of the electron donor compound of formula (I) is such that its molar ratio with respect to the Ti atoms is from 0.01 to 2, preferably from 0.05 to 1.5.

The solid catalyst components according to the present disclosure are converted into catalysts for the polymerization of olefins by reacting them with organoaluminum compounds according to known methods.

In particular, it is an object of the present disclosure a catalyst for the polymerization of olefins CH₂=CHR, in which R is hydrogen or a hydrocarbyl radical with 1-12 carbon atoms, comprising the product obtained by contacting:
(i) the solid catalyst component as disclosed above and
(ii) an alkylaluminum compound and optionally,
(iii) an external electron donor compound.

The alkyl-Al compound (ii) is preferably chosen among the trialkyl aluminum compounds such as for example triethylaluminum, triisobutylaluminum, tri-n-butylaluminum, tri-n-hexylaluminum, tri-n-octylaluminum. It is also possible to use alkylaluminum halides, alkylaluminum hydrides or alkylaluminum sesquichlorides, such as AlEt₂Cl and Al₂Et₃Cl₃, possibly in mixture with the above cited trialkylaluminums.

Suitable external electron-donor compounds include silicon compounds, ethers, esters, amines, heterocyclic compounds and particularly 2,2,6,6-tetramethylpiperidine and ketones.

Another class of preferred external donor compounds is that of silicon compounds of formula (R₇)ₐ(R₈)_{b}Si(OR₉)_{c}, where a and b are integers from 0 to 2, c is an integer from 1 to 4 and the sum (a+b+c) is 4; R₇, R₈, and R₉, are radicals with 1-18 carbon atoms optionally containing heteroatoms. Particularly preferred are the silicon compounds in which a is 1, b is 1, c is 2, at least one of R₇ and R₈ is selected from branched alkyl, cycloalkyl or aryl groups with 3-10 carbon atoms optionally containing heteroatoms and R₉ is a C₁-C₁₀ alkyl group, in particular methyl. Examples of such preferred silicon compounds are methylcyclohexyldimethoxysilane (C donor), diphenyldimethoxysilane, methyl-t-butyldimethoxysilane, dicyclopentyldimethoxysilane (D donor), diisopropyldimethoxysilane, (2-ethylpiperidinyl)t-butyldimethoxysilane, (2-ethylpiperidinyl)thexyldimethoxysilane, (3,3,3-trifluoro-n-propyl)(2-ethylpiperidinyl)dimethoxysilane, methyl(3,3,3-trifluoro-n-propyl)dimethoxysilane, N,N-diethylaminotriethoxysilane. Moreover, are also preferred the silicon compounds in which a is 0, c is 3, R₈ is a branched alkyl or cycloalkyl group, optionally containing heteroatoms, and R₉ is methyl. Examples of such preferred silicon compounds are cyclohexyltrimethoxysilane, t-butyltrimethoxysilane and thexyltrimethoxysilane.

The electron donor compound (iii) is used in such an amount to give a molar ratio between the organoaluminum compound and said electron donor compound (iii) of from 0.1 to 500, preferably from 1 to 300 and more preferably from 3 to 100.

Therefore, it constitutes a further object of the present disclosure a process for the (co)polymerization of olefins CH₂=CHR, in which R is hydrogen or a hydrocarbyl radical with 1-12 carbon atoms, carried out in the presence of a catalyst comprising the product of the reaction between:
(i) the solid catalyst component of the disclosure;
(ii) an alkylaluminum compound and,
(iii) optionally an electron-donor compound (external donor).

The polymerization process can be carried out according to known techniques for example slurry polymerization using as diluent an inert hydrocarbon solvent, or bulk polymerization using the liquid monomer (for example propylene) as a reaction medium. Moreover, it is possible to carry out the polymerization process in gas-phase operating in one or more fluidized or mechanically agitated bed reactors.

The polymerization is generally carried out at temperature of from 20 to 120°C, preferably of from 40 to 80°C. When the polymerization is carried out in gas-phase the operating pressure is generally between 0.5 and 5 MPa, preferably between 1 and 4 MPa. In the bulk polymerization the operating pressure is generally between 1 and 8 MPa, preferably between 1.5 and 5 MPa.

In the polymerization of propylene using the catalyst of the present disclosure and under the standard polymerization conditions described in the experimental section it is possible to obtain polymerization activities ranging from 50 to 90 kg/g cat. Combined with high polymer isotacticity expressed by a value of xylene insoluble matter of higher than 98.5% preferably higher than 99%wt.

The following examples are given in order to further illustrate the disclosure.

### CHARACTERIZATIONS

### Determination of X.I.

2.5 g of polymer and 250 ml of o-xylene were placed in a round-bottomed flask provided with a cooler and a reflux condenser and kept under nitrogen. The obtained mixture was heated to 135°C and was kept under stirring for about 60 minutes. The final solution was allowed to cool to 25°C under continuous stirring, and the insoluble polymer was then filtered. The filtrate was then evaporated in a nitrogen flow at 140°C to reach a constant weight. The content of said xylene-soluble fraction is expressed as a percentage of the original 2.5 grams and then, by difference, the X.I. %.

### Determination of donors.

The content of electron donor has been carried out via gas-chromatography. The solid component was dissolved in acidic water. The solution was extracted with ethyl acetate, an internal standard was added, and a sample of the organic phase was analyzed in a gas chromatograph, to determine the amount of donor present at the starting catalyst compound.

### Melt flow rate (MFR)

The melt flow rate MIL of the polymer was determined according to ISO 1133 (230°C, 2.16 Kg).

### EXAMPLES

### Synthetic Example 1

### Mixture of 4-(tert-butyl)-2-((ethoxycarbonyl)oxy)-6-methylphenyl diethylcarbamate and 5-(tert-butyl)-2-((ethoxycarbonyl)oxy)-3-methylphenyl diethylcarbamate.

First step: In a 500 cm³ round bottom flask, under nitrogen, are introduced THF (240 cm³), 3-methyl-5-tert-butyl-catechol (30 g, 0.166 mol), triethylamine (51 cm3, 0.366 mol) and dichloroacetyl chloride (33.7 cm³, 0.350 mol) is added dropwise with cooling. After 1 hour at room temperature the mixture is quenched with aqueous HCl, extracted with ethyl acetate and washed with water until neutral pH, then is anhydrified over Na₂SO₄ and the solvent is distilled off to afford 67 g of white solid which is pure 5-(tert-butyl)-3-methyl-1,2-phenylene bis(2,2-dichloroacetate). Yield 99%

Second step: In a 1 dm³ round bottom flask, under nitrogen, is charged THF (330 cm³), NaH (7.85 g, 0.327 mol) and a solution of 5-(tert-butyl)-3-methyl-1,2-phenylene bis(2,2-dichloroacetate (62.6 g, 0.156 mol) dissolved in THF (50 cm³) is added dropwise at room temperature. The dark brown mixture is stirred at room temperature until GC shows that the reaction is completed (43 hours). Then the mixture is quenched with water, diethyl ether is added and the organic layer is washed with aqueous NaHCO₃, then is anhydrified over Na₂SO₄ and the solvent is distilled off to afford a dark brown crude which is distilled at reduced pressure. 25.1 g of a yellow solid, which constists of pure 6-(tert-butyl)-4-methylbenzo[d][1,3]dioxol-2-one is obtained. Yield 77%.

Third step: In a 500 cm³ round bottom flask, under nitrogen, are introduced THF (175 cm³) and 6-(tert-butyl)-4-methylbenzo[d][1,3]dioxol-2-one (25 g, 0.117 mol). The mixture is cooled down to -5°C, then diethylamine is added dropwise (6.9 cm³, 0.330 mol). Then the mixture is brought to room temperature and after one hour of stirring are added sequentially, with cooling, triethylamine (14.7 cm³, 0.145 mol) and ethyl chloroformate (18 cm³, 0.145 mol). After one hour the reaction is quenched with aqueous HCl, extracted with ethyl acetate and washed with water until neutral pH, then is anhydrified over Na₂SO₄ and the solvent is distilled off to afford 41.2 of a mixture of 4-(tert-butyl)-2-((ethoxycarbonyl)oxy)-6-methylphenyl diethylcarbamate and 5-(tert-butyl)-2-((ethoxycarbonyl)oxy)-3-methylphenyl diethylcarbamate. Yield 96%.

### Synthetic Example 2

### Mixture of 4-(tert-butyl)-2-((ethoxycarbonyl)oxy)-6-methylphenyl dipropylcarbamate and 5-(tert-butyl)-2-((ethoxycarbonyl)oxy)-3-methylphenyl dipropylcarbamate.

The procedure is the same as that of Synthetic Example 1 except that dipropylamine is used instead of diethylamine in the third step.

### Synthetic Example 3

### Mixture of 2-((ethoxycarbonyl)oxy)-3-methylphenyl diethylcarbamate and 2-((ethoxycarbonyl)oxy)-6-methylphenyl diethylcarbamate.

The procedure is the same of Synthetic Example 1 except that 3-methylcatechol is used instead of 3-methyl-5-tert-butyl-catechol in the first step.

### Comparative Synthetic Example 4

### 5-(tert-butyl)-3-methyl-1,2 phenylene diethyl dicarbonate

The synthesis of 5-(tert-butyl)-3-methyl-1,2-phenylene diethyl dicarbonate has been done according to the general preparation described in US2011/0130530.

### Synthetic Example 5

### Mixture of 2-((butoxycarbonyl)oxy)-4-(tert-butyl)-6-methylphenyl diethylcarbamate and 2-((butoxycarbonyl)oxy)-5-(tert-butyl)-3-methylphenyl diethylcarbamate.

The procedure is the same as that of Synthetic Example 1 except that butyl chloroformate is used instead of ethyl chloroformate in the third step.

### Synthetic Example 6

### Mixture of 4-(tert-butyl)-2-((isobutoxycarbonyl)oxy)-6-methylphenyl diethylcarbamate and 5-(tert-butyl)-2-((isobutoxycarbonyl)oxy)-3-methylphenyl diethylcarbamate.

The procedure is the same as that of Synthetic Example 1 except that isobutyl chloroformate is used instead of ethyl chloroformate in the third step.

### Synthetic Example 7

### Mixture of 2-((ethoxycarbonyl)oxy)-3-methylphenyl dipropylcarbamate and 2-((ethoxycarbonyl)oxy)-6-methylphenyl dipropylcarbamate.

The procedure is the same of Synthetic Example 1 except that 3-methylcatechol is used instead of 3-methyl-5-tert-butyl-catechol in the first step and dipropylamine is used instead of diethylamine in the third step.

### Synthetic Example 8

### Mixture of 4-(tert-butyl)-2-((ethoxycarbonyl)oxy)phenyl diethylcarbamate and 5-(tert-butyl)-2-((ethoxycarbonyl)oxy)phenyl diethylcarbamate.

The procedure is the same of Synthetic Example 1 except that 4-tert-butyl-catechol is used instead of 3-methyl-5-tert-butyl-catechol in the first step.

### General procedure for preparation of the spherical adducts A and B

An initial amount of microspheroidal MgCl₂.2.8C₂H₅OH was prepared according to the method described in Example 2 of WO98/44009, but operating on larger scale. This adduct is called adduct A. The solid adduct A was then subject to thermal dealcoholation at increasing temperatures from 30 to 130°C and operating in nitrogen current until reaching an alcohol content of 1.9 moles per mol of MgCl₂. This partially dealcoholated adduct is called adduct B.

### General procedure for the polymerization of propylene

A 4-litre steel autoclave equipped with a stirrer, pressure gauge, thermometer, catalyst feeding system, monomer feeding lines and thermostating jacket, was purged with nitrogen flow at 70°C for one hour. Then, at 30°C under propylene flow, were charged in sequence with 75 mL of anhydrous hexane, 0.76 g of AlEt₃, the external electron donor indicated in Table 1 (if used) and 0.006÷0.010 g of solid catalyst component. The autoclave was closed; subsequently 2.0 NL of hydrogen were added. Then, under stirring, 1.2 kg of liquid propylene was fed. The temperature was raised to 70°C in five minutes and the polymerization was carried out at this temperature for two hours. At the end of the polymerization, the non-reacted propylene was removed; the polymer was recovered and dried at 70°C under vacuum for three hours. Then the polymer was weighed and fractionated with o-xylene to determine the amount of the xylene insoluble (X.I.) fraction.

### EXAMPLE 1

### Preparation of Solid Catalyst Component and polymerization

Into a 500 cm³ round bottom flask, equipped with mechanical stirrer, cooler and thermometer 250 cm³ of TiCl₄ were introduced at room temperature under nitrogen atmosphere. After cooling to 0°C, while stirring, the internal donor of synthetic example 1 and 10.0 g of the spherical adduct A were sequentially added into the flask. The amount of charged internal donor was such to charge a Mg/donor molar ratio of 6. The temperature was raised to 100°C and maintained for 2 hours. Thereafter, stirring was stopped, the solid product was allowed to settle and the supernatant liquid was siphoned off at 100°C. After the supernatant was removed, additional fresh TiCl₄ was added to reach the initial liquid volume again. The mixture was then heated at 120°C and kept at this temperature for 1 hour. Stirring was stopped again, the solid was allowed to settle and the supernatant liquid was siphoned off. The solid was washed with anhydrous hexane six times (6 x 100 cm³) in temperature gradient down to 60°C and one time (100 cm³) at room temperature. The obtained solid was then dried under vacuum and analyzed. The obtained solid catalyst component contains 4.3%wt of Ti, and 18.0 %wt of internal donor. The so obtained solid catalyst components were tested in polymerization of propylene, using the procedure described above. The results are listed in Table 1.

### EXAMPLE 2

### Preparation of Solid Catalyst Component and polymerization

The procedure described above for solid catalyst component 1 was repeated, using the donor of synthetic example 2 as internal donor. The so obtained solid catalyst components were tested in polymerization of propylene, using the procedure described above. The results are listed in Table 1.

### EXAMPLE 3

### Preparation of Solid Catalyst Component and polymerization

The procedure described above for solid catalyst component 1 was repeated, using the donor prepared in synthetic example 3. The so obtained solid catalyst components were tested in polymerization of propylene, using the procedure described above. The results are listed in Table 1.

### EXAMPLE 4

### Preparation of Solid Catalyst Component and polymerization

Into a 500 cm³ round bottom flask, equipped with mechanical stirrer, cooler and thermometer 250 cm³ of TiCl₄ were introduced at room temperature under nitrogen atmosphere. After cooling to 0°C, while stirring, the internal donor prepared in synthetic example 1 and 10.0 g of the spherical adduct B were sequentially added into the flask. The amount of charged internal donor was such to charge a Mg/donor molar ratio of 8. The temperature was raised to 120°C and maintained for 1 hour. Thereafter, stirring was stopped, the solid product was allowed to settle and the supernatant liquid was siphoned off at 120°C. After the supernatant was removed, additional fresh TiCl₄ was added to reach the initial liquid volume again. The mixture was then heated at 120°C and kept at this temperature for 30 minutes. Stirring was stopped again, the solid was allowed to settle and the supernatant liquid was siphoned off. After the supernatant was removed, additional fresh TiCl₄ was added to reach the initial liquid volume again. The mixture was then heated at 120°C and kept at this temperature for 15 minutes. Stirring was stopped again, the solid was allowed to settle and the supernatant liquid was siphoned off. The obtained solid was washed four times with anhydrous heptane (4 x 100 cm³) at 90°C and two times with anhydrous iso-hexane (2 x 100 cm³) at 25°C then dried under vacuum. The obtained solid catalyst component contains 3.2 %wt of Ti, and 14.4%wt of internal donor. The so obtained solid catalyst components were tested in polymerization of propylene, using the procedure described above. The results are listed in Table 1.

### EXAMPLES 5-8

The procedure described above for solid catalyst component 1 was repeated, using the donors prepared in synthetic examples 5-8 respectively. The so obtained solid catalyst components were tested in polymerization of propylene, using the procedure described above. The results and the catalyst composition is reported Table 1.

### COMPARATIVE EXAMPLE 1

### Preparation of Solid Catalyst Component and polymerization

The procedure described above for solid catalyst component 1 was repeated, using the donor prepared according to comparative synthetic example 4 as internal donor. The obtained solid catalyst component contains 2.6 %wt of Ti, and 20.7 %wt of internal donor. The so obtained solid catalyst components were tested in polymerization of propylene, using the procedure described above. The results are listed in Table 1.

### COMPARATIVE EXAMPLE 2

Preparation of Solid Catalyst Component 6 (ID= N-cbz-L-proline methyl ester)

The procedure described above for solid catalyst component 1 was repeated, using commercially available (Sigma-Aldrich) N-cbz-L-proline methyl ester. The obtained solid catalyst component contains 5.4 %wt of Ti. The so obtained solid catalyst components were tested in polymerization of propylene, using the procedure described above. The results are listed in Table 1.

**TABLE 1**

| **EX** | **Catalyst composition** | | **Polymerization** | | | |
|---|---|---|---|---|---|---|
| | ID%wt | Ti%wt | ED | Activity | XI | MIL |
| | | | | **kg/g** | **%wt** | **g/10'** |
| 1 | 18 | 4.3 | D | 75 | 99.2 | 0.5 |
| | " | " | C | 82 | 98.9 | 0.3 |
| | " | " | No ED | 97 | 96.3 | 0.4 |
| 2 | 14.5 | 4 | D | 75 | 99.2 | 0.6 |
| 3 | 17.2 | 3.6 | D | 50 | 98.3 | 1.5 |
| 4 | 14.4 | 3.2 | D | 81 | 98.8 | 0.9 |
| | " | " | C | 80 | 98.7 | 0.4 |
| | " | " | No ED | 122 | 94.4 | 1.4 |
| C1 | 20.7 | 2.6 | D | 36 | 98.5 | 3.6 |
| C2 | n.d | 5.4 | D | 13 | 93.1 | 6.9 |
| 5 | n.d. | 4.4 | D | 76 | 98.9 | 0.8 |
| 6 | n.d. | 4.6 | D | 58 | 98.6 | 0.7 |
| 7 | n.d. | 3.9 | D | 51 | 98.1 | 2.8 |
| 8 | 17.9 | 3.2 | D | 59 | 98.9 | 0.4 |

| | | | | | | |
|---|---|---|---|---|---|---|
| ED: External Donor D: Dicyclopentyldimethoxysilane C: Methylcyclohexyldimethoxysilane | | | | | | |

## Claims

1. A solid catalyst component for the polymerization of olefins comprising Mg, Ti and an electron donor of formula (I) 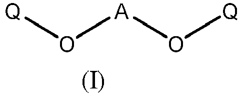
where each Q is a group -COOR¹ in which R¹ is selected from C₁-C₁₅ hydrocarbon groups, optionally containing a heteroatom selected from halogen, P, S, N, O; or a
group -CON(R²)₂ in which R² groups, equal to or different from each other, are hydrogen or R¹ groups which can be fused together to form one or more cycles, and A is a bivalent bridging group with the proviso that Q groups cannot be simultaneously a group -COOR¹ or -CON(R²)₂.

2. The catalyst component according to claim 1 in which A is bivalent bridging group with chain length between the two free radicals being 1-10 atoms.

3. The catalyst component according to claim 1 in which the bridging group has formula -(ZR³ₘ)ₙ- in which, independently, Z is selected from C, Si, Ge, O, N, S or P, the R³ groups, equal to or different from each other, are hydrogen or C₁-C₂₀ hydrocarbon radicals, optionally containing a heteroatom selected from halogen, P, S, N, O and Si, which can be fused together to form one or more cycles, m is a number satisfying the valences of Z and n is an integer ranging from 1 to 10.

4. The catalyst component according to claim 3 in which bridging group is the one based on aromatic groups which through the carbon ring atoms can link the two oxygen of formula (I).

5. The catalyst component according to claim 4 in which the bridging group is selected form phenyl groups, optionally substituted with halogens or C₁-C₂₀ alkyl radicals, bridging the oxygen atoms in position 1,2 or 1,3 or 1,4 and the naphthalene groups, optionally substituted bridging the oxygen groups in position 1,2 or 2,3 or 1,8.

6. The catalyst component according to claim 1 in which the donor is selected from the compounds of formula (II) 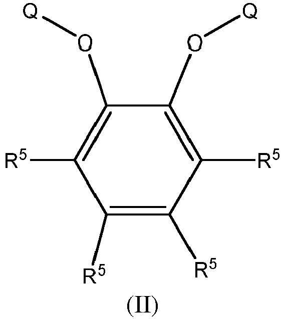 in which Q has the same meaning as in claim 1 and R⁵, independently, is selected from hydrogen, halogens or C₁-C₁₅ hydrocarbon groups optionally substituted with heteroatoms selected from halogen, P, S, N, O and Si, with the proviso that at least one of R⁵ is different from hydrogen.

7. The catalyst component according to claim 6 in which at least two of the R⁵ groups are different from hydrogen.

8. The catalyst component according to claim 7 in which the aromatic ring of formula (II) is substituted in position 3,5 with R⁵ groups are selected from C₁-C₅ alkyl groups.

9. The catalyst component according to any of the preceding claims in which R¹ and R² groups independently selected from C₁-C₁₅ alkyl groups, C₆-C₁₄ aryl groups, C₃-C₁₅ cycloalkyl groups, and C₇-C₁₅ arylalkyl or alkylaryl groups; with the proviso that R² groups can also be hydrogen.

10. The catalyst component according to claim 6 in which the donor is selected from the compounds of formula (III) 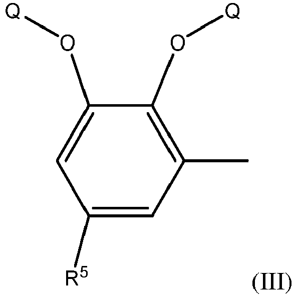 in which Q has the same meaning as in claim 1 and R⁵ is a branched alkyl group.

11. The catalyst component according to claim 10 in which R¹ and R² are C₁-C₅ alkyl groups and R⁵ is t-butyl.

12. A catalyst for the polymerization of olefins comprising the product of the reaction between:
(i) the solid catalyst component according to any of the preceding claims and
(ii) an alkylaluminum compound and optionally,
(iii) an external electron donor compound.

13. The catalyst according to claim 12 in which the external donor is selected from silicon compounds of formula (R₇)ₐ(R₈)_{b}Si(OR₉)_{c}, where a and b are integers from 0 to 2, c is an integer from 1 to 4, the sum (a+b+c) is 4 and R₇, R₈, and R₉ are radicals with 1-18 carbon atoms optionally containing heteroatoms.

14. A process for the (co)polymerization of olefins CH₂=CHR, in which R is hydrogen or a hydrocarbyl radical with 1-12 carbon atoms, carried out in the presence of a catalyst system comprising the product of the reaction between:
i. the solid catalyst component according to any of the proceeding claims;
ii. an alkylaluminum compound and,
iii. optionally an external donor compound.

## Patentansprüche

1. Feste Katalysatorkomponente für die Polymerisation von Olefinen, umfassend Mg, Ti und einen Elektronendonor mit der Formel (I) 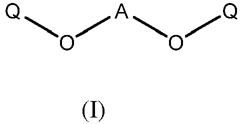
wobei jedes Q eine Gruppe -COOR¹, in der R¹ ausgewählt ist aus C₁-C₁₅-Kohlenwasserstoffgruppen, die gegebenenfalls ein Heteroatom ausgewählt aus Halogen, P, S, N, O enthalten; oder
eine Gruppe -CON(R²)₂ ist, in der die Gruppen R², die gleich oder voneinander verschieden sind, Wasserstoff oder Gruppen R¹ sind, die unter Bildung von einem Cyclus oder mehreren Cyclen kondensiert sein können, und
A eine zweiwertige Brückengruppe ist, mit der Maßgabe, dass die Gruppen Q nicht gleichzeitig eine Gruppe -COOR¹ oder -CON(R²)₂ sein können.

2. Katalysatorkomponente nach Anspruch 1, wobei A eine zweiwertige Brückengruppe mit einer Kettenlänge zwischen den beiden freien Resten von 1-10 Atomen ist.

3. Katalysatorkomponente nach Anspruch 1, wobei die Brückengruppe die Formel -(ZR³ₘ)ₙ- hat, in der Z unabhängig ausgewählt ist aus C, Si, Ge, O, N, S oder P, die Gruppen R³ gleich oder voneinander verschieden sind und Wasserstoff oder C₁-C₂₀-Kohlenwasserstoffreste sind, die gegebenenfalls ein Heteroatom ausgewählt aus Halogen, P, S, N, O und Si enthalten, die miteinander unter Bildung von einem Cyclus oder mehreren Cyclen kondensiert sein können, m eine Zahl ist, die die Wertigkeiten von Z absättigt, und n eine ganze Zahl im Bereich von 1 bis 10 ist.

4. Katalysatorkomponente nach Anspruch 3, wobei die Brückengruppe diejenige auf Basis von aromatischen Gruppen ist, die über die Kohlenstoffringatome die beiden Sauerstoffe der Formel (I) verknüpfen können.

5. Katalysatorkomponente nach Anspruch 4, wobei die Brückengruppe ausgewählt ist aus Phenylgruppen, die gegebenenfalls mit Halogenen oder C₁-C₂₀-Alkylresten substituiert sind, die die Sauerstoffatome in Position 1,2 oder 1,3 oder 1,4 verbrücken, und Naphthalingruppen, die gegebenenfalls substituiert sind, die die Sauerstoffgruppen in Position 1,2 oder 2,3 oder 1,8 verbrücken.

6. Katalysatorkomponente nach Anspruch 1, wobei der Donor ausgewählt ist aus Verbindungen mit der Formel (II) 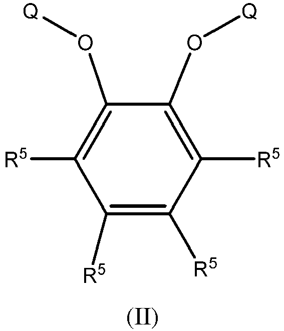 wobei Q die gleiche Bedeutung wie in Anspruch 1 hat, und R⁵ unabhängig ausgewählt ist aus Wasserstoff, Halogenen oder C₁-C₁₅-Kohlenwasserstoffgruppen, die gegebenenfalls mit Heteroatomen substituiert sind, die ausgewählt sind aus Halogen, P, S, N, O und Si, mit der Maßgabe, dass mindestens eines von R⁵ von Wasserstoff verschieden ist.

7. Katalysatorkomponente nach Anspruch 6, wobei sich mindestens zwei der Gruppen R⁵ von Wasserstoff unterscheiden.

8. Katalysatorkomponente nach Anspruch 7, wobei der aromatische Ring der Formel (II) in Position 3,5 mit Gruppen R⁵ substituiert ist, die ausgewählt sind aus C₁-C₅-Alkylgruppen.

9. Katalysatorkomponente nach einem der vorhergehenden Ansprüche, wobei die Gruppen R¹ und R² unabhängig ausgewählt sind aus C₁-C₁₅-Alkylgruppen, C₆-C₁₄-Arylgruppen, C₃-C₁₅-Cycloalkylgruppen und C₇-C₁₅-Arylalkyl- oder -Alkylarylgruppen, mit der Maßgabe, dass die Gruppen R² auch Wasserstoff sein können.

10. Katalysatorkomponente nach Anspruch 6, wobei der Donor ausgewählt ist aus den Verbindungen der Formel (III): 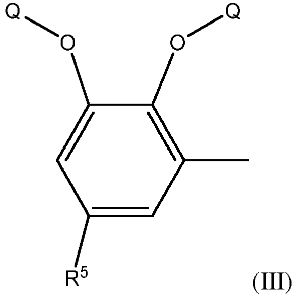 wobei Q die gleiche Bedeutung wie in Anspruch 1 hat und R⁵ eine verzweigte Alkylgruppe ist.

11. Katalysatorkomponente nach Anspruch 10, wobei R¹ und R² C₁-C₅-Alkylgruppen sind, und R⁵ t-Butyl ist.

12. Katalysator zur Polymerisation von Olefinen, umfassend das Produkt der Reaktion zwischen:
(i) der festen Katalysatorkomponente gemäß einem der vorhergehenden Ansprüche und
(ii) einer Alkylaluminiumverbindung und gegebenenfalls
(iii) einer externen Elektronendonorverbindung.

13. Katalysator nach Anspruch 12, wobei der externe Donor ausgewählt ist aus Siliciumverbindungen der Formel (R₇)ₐ(R₈)_{b}Si(OR₉)_{c}, wobei a und b ganze Zahlen von 0 bis 2 sind, c eine ganze Zahl von 1 bis 4 ist und die Summe (a+b+c) 4 ist; und R₇, R₈ und R₉ Reste mit 1 bis 18 Kohlenstoffatomen sind, die gegebenenfalls Heteroatome enthalten.

14. Verfahren zur (Co)polymerisation von Olefinen CH₂=CHR, wobei R Wasserstoff oder ein Kohlenwasserstoffrest mit 1-12 Kohlenstoffatomen ist, welches in Gegenwart eines Katalysatorsystems durchgeführt wird, das das Produkt der Reaktion zwischen:
i. der festen Katalysatorkomponente gemäß einem der vorhergehenden Ansprüche;
ii. einer Alkylaluminiumverbindung und
iii. gegebenenfalls einer externen Donorverbindung umfasst.

## Revendications

1. Composant catalyseur solide pour la polymérisation d'oléfines comprenant Mg, Ti et un donneur d'électrons de formule (I) 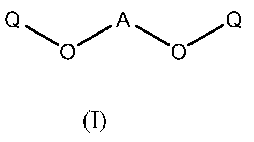 où chaque Q est un groupe -COOR¹ dans lequel R¹ est choisi parmi des groupes hydrocarbonés en C₁ à C₁₅, contenant éventuellement un hétéroatome choisi parmi halogène, P, S, N, O ; ou un groupe -CON(R²)₂ dans lequel les groupes R², égaux ou différents les uns par rapport aux autres, sont l'hydrogène ou des groupes R¹ qui peuvent être condensés ensemble pour former un ou plusieurs cycles, et A est un groupe de pontage bivalent à condition que les groupes Q ne puissent pas être simultanément un groupe -COOR¹ ou -CON(R²)₂.

2. Composant catalyseur selon la revendication 1, dans lequel A est un groupe de pontage bivalent avec une longueur de chaîne entre les deux radicaux libres allant de 1 à 10 atomes.

3. Composant catalyseur selon la revendication 1, dans lequel le groupe de pontage est de formule -(ZR³ₘ)ₙ- dans laquelle, indépendamment, Z est choisi parmi C, Si, Ge, O, N, S ou P, les groupes R³, égaux ou différents les uns par rapport aux autres, sont l'hydrogène ou des radicaux hydrocarbonés en C₁ à C₂₀, contenant éventuellement un hétéroatome choisi parmi halogène, P, S, N, O et Si, qui peuvent être condensés ensemble pour former un ou plusieurs cycles, m est un nombre satisfaisant aux valences de Z et n est un nombre entier allant de 1 à 10.

4. Composant catalyseur selon la revendication 3, dans lequel le groupe de pontage est celui à base de groupes aromatiques qui, par le biais des atomes de cycle carbone, peut lier les deux oxygènes de la formule (I).

5. Composant catalyseur selon la revendication 4, dans lequel le groupe de pontage est choisi parmi des groupes phényle, éventuellement substitués par des halogènes ou des radicaux alkyle en C₁ à C₂₀, pontant les atomes d'oxygène en position 1,2 ou 1,3 ou 1,4 et les groupes naphtalène, éventuellement substitués pontant les groupes oxygène en position 1,2 ou 2,3 ou 1,8.

6. Composant catalyseur selon la revendication 1, dans lequel le donneur est choisi parmi les composés de formule (II) 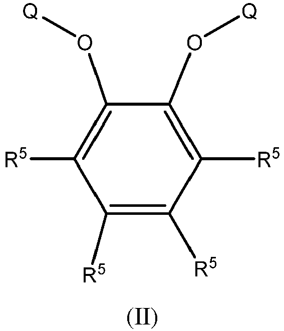 dans laquelle Q a la même signification que dans la revendication 1 et R⁵, indépendamment, est choisi parmi l'hydrogène, des halogènes ou des groupes hydrocarbonés en C₁ à C₁₅ éventuellement substitués par des hétéroatomes choisis parmi halogène, P, S, N, O et Si, à condition qu'au moins l'un des R⁵ soit différent de l'hydrogène.

7. Composant catalyseur selon la revendication 6, dans lequel au moins deux des groupes R⁵ sont différents de l'hydrogène.

8. Composant catalyseur selon la revendication 7, dans lequel le cycle aromatique de formule (II) est substitué en position 3,5 avec des groupes R⁵ qui sont choisis parmi des groupes alkyle en C₁ à C₅*.*

9. Composant catalyseur selon l'une quelconque des revendications précédentes, dans lequel les groupes R¹ et R² sont indépendamment choisis parmi des groupes alkyle en C₁ à C₁₅, des groupes aryle en C₆ à C₁₄, des groupes cycloalkyle en C₃ à C₁₅ et des groupes arylalkyle ou alkylaryle en C₇ à C₁₅ ; à condition que les groupes R² puissent également être l'hydrogène.

10. Composant catalyseur selon la revendication 6, dans lequel le donneur est choisi parmi les composés de formule (III) 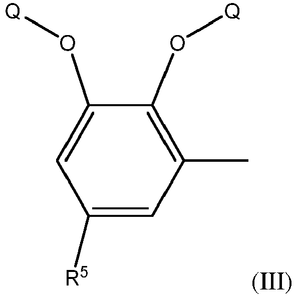 dans laquelle Q a la même signification que dans la revendication 1 et R⁵ est un groupe alkyle ramifié.

11. Composant catalyseur selon la revendication 10, dans lequel R¹ et R² sont des groupes alkyle en C₁ à C₅ et R⁵ est t-butyle.

12. Catalyseur pour la polymérisation d'oléfines, comprenant le produit de la réaction entre :
(i) le composant catalyseur solide selon l'une quelconque des revendications précédentes et
(ii) un composé alkylaluminium et, éventuellement,
(iii) un composé donneur d'électrons externe.

13. Catalyseur selon la revendication 12, dans lequel le donneur externe est choisi parmi des composés de silicium de formule (R₇)ₐ(R₈)_{b}Si(OR₉)_{c}, où a et b sont des nombres entiers allant de 0 à 2, c est un nombre entier allant de 1 à 4, la somme (a+b+c) vaut 4 et R₇, R₈ et R₉ sont des radicaux avec 1 à 18 atomes de carbone contenant éventuellement des hétéroatomes.

14. Procédé de (co)polymérisation d'oléfines CH₂=CHR, dans lequel R est un hydrogène ou un radical hydrocarbyle avec 1 à 12 atomes de carbone, effectué en présence d'un système de catalyseur comprenant le produit de la réaction entre :
i. le composant catalyseur solide selon l'une quelconque des revendications précédentes ;
ii. un composé alkylaluminium et,
iii. éventuellement un composé donneur externe.
